# EUROPEAN PATENT APPLICATION

(11) **EP 4 393 458 A1**
(43) Date of publication of application: **03.07.2024**
(21) Application number: 22923176.6
(22) Date of filing: 29.06.2022
(51) Int. Cl.: A61F 13/02, A61M 37/00, A61B 5/145

(54) **INTEGRATED BALANCED PLASTER TYPE LIQUID DRUG INFUSION SYSTEM**

(30) Priority: 25.01.2022 CN 202210088376
(71) Applicant: Aaruy Medical Electronics Co., Ltd., Shenzhen, Guangdong 518000 (CN)
(72) Inventor: ZHOU, Qingxu, Shenzhen, Guangdong 518000 (CN)
(74) Representative: Petraz, Gilberto Luigi
(86) International application number: PCT/CN2022/102228
(87) International publication number: WO 2023/142368

(57) **Abstract**

Provided in the present application is an integrated balanced paste-type medicine infusion system by including an injection device, a medicine delivery device, a baseplate, an adhesive member, a main body shell and a controlling device; the controlling device is mounted in the main body shell; the injection device, the medicine delivery device and the main body shell are mounted on the baseplate; the adhesive member is fixedly provided on a side of the baseplate distal to the main body shell; then the adhesive member is used to attach the baseplate to skin; the medicine delivery device is used to store medicinal liquid; an output end of the medicine delivery device is connected to an input end of the injection device; the controlling device is used to control the medicine delivery device to output the medicinal liquid to the injection device; the main body shell is provided with a first avoidance hole penetratingly; the output end of the medicine delivery device is mounted on the first avoidance hole; the baseplate is provided with a second avoidance hole penetratingly; the injection device is mounted on the second avoidance hole; and the second avoidance hole is fitted with the first avoidance hole. Provided in the embodiments of the present application, when separating the medicine delivery device 2, the controlling device, and the injection device 1, it is unnecessary to disassemble the main body shell 4 and simple to operate.

## Description

### Field of the invention

The present application relates to the field of medical devices, in particular to an integrated balanced paste-type medicine infusion system.

### Background of the invention

Current paste-type medicine infusion system is commonly used in the treatment of diseases such as diabetes. Medicinal liquid, such as insulin, is stored in the paste-type medicine infusion system. The paste-type medicine infusion system may be attached to a surface of the skin of humans or other living organisms, which may inject medicinal liquid into the body of humans or other living organisms at any time and any place as demand.

The paste-type medicine infusion system usually includes an injection device, a medicine delivery device, a baseplate, a main body shell and a controlling device, in which the injection device, the medicine delivery device and the controlling device are all mounted in the main body shell. The main body shell is provided on the baseplate. The baseplate is attached to a surface of the skin of humans or other living organisms by applying adhesive members such as an adhesive tape.

Since the injection device, the medicine delivery device, and the controlling device are all provided in the main body shell, when needing to separate the medicine delivery device, the controlling device, and the injection device, it is necessary to disassemble the main body shell and complicated to operate.

### Summary of the invention

Provided in the present application is an integrated balanced paste-type medicine infusion system for providing a paste-type medicine infusion system that facilitates the separation of a medicine delivery device, a controlling device, and an injection device;
Provided in the present application is an integrated balanced paste-type medicine infusion system by including an injection device, a medicine delivery device, a baseplate, an adhesive member, a main body shell and a controlling device;
the controlling device is mounted in the main body shell; the injection device, the medicine delivery device and the main body shell are mounted on the baseplate; the adhesive member is fixedly provided on a side of the baseplate distal to the main body shell; then the adhesive member is used to attach the baseplate to skin;
the medicine delivery device is used to store medicinal liquid; an output end of the medicine delivery device is connected to an input end of the injection device; the controlling device is used to control the medicine delivery device to output the medicinal liquid to the injection device;
the main body shell is provided with a first avoidance hole penetratingly; the output end of the medicine delivery device is mounted on the first avoidance hole;
the baseplate is provided with a second avoidance hole penetratingly; the injection device is mounted on the second avoidance hole; and the second avoidance hole is fitted with the first avoidance hole.

In an embodiment of the present application, the main body shell is provided with a first avoidance hole penetratingly; the baseplate is provided with a second avoidance hole penetratingly; and the second avoidance hole is fitted with the first avoidance hole, so that the first avoidance hole is in communication with the second avoidance hole in the state of use. The output end of the medicine delivery device is mounted in the first avoidance hole and the injection device is mounted in the second avoidance hole, the output end of the medicine delivery device is connected to the injection device. The controlling device is mounted in the main body shell, while the medicine delivery device and the injection device are mounted out of the main body shell. When separating the medicine delivery device, the controlling device, and the injection device, it is unnecessary to disassemble the main body shell and simple to operate.

In one implementation of an embodiment of the present application, the second avoidance hole is provided in the central predetermined area of the baseplate.

In an embodiment of the present application, the second avoidance hole is provided in a central predetermined area of the baseplate, so that the injection device is provided in a central position of the entire integrated balanced paste-type medicine infusion system. Compared to providing the second avoidance hole in an edge area of the baseplate, there is less movement of the injection device relative to the skin, which reduces skin damage caused by a large movement of the injection device.

In one implementation of an embodiment of the present application, the output end of the medicine delivery device is inserted into the first avoidance hole from a side of the main body shell distal to the baseplate; the injection device is mounted in the second avoidance hole of the baseplate; and the injection device is inserted into the first avoidance hole from a side of the main body shell proximal to the baseplate.

In an embodiment of the present application, it is convenient to disassemble and assemble the medicine delivery device, the controlling device and the injection device by inserting the output end of the medicine delivery device and the injection device respective from both sides of the first avoidance hole.

In one implementation of an embodiment of the present application, the injection device includes an indwelling needle and a needle holder, and the needle holder is used to mount the indwelling needle to the baseplate.

In an embodiment of the present application, the indwelling needle may be mounted on the baseplate more stably and conveniently by adopting the needle holder. The needle holder may be fitted with the output end of the medicine delivery device, facilitating the connection and disassembly of the injection device and the output end of the medicine delivery device.

In one implementation of an embodiment of the present application, the main body shell is provided with a mounting buckle in the first avoidance hole, and the mounting buckle is used to snap-fit the output end of the medicine delivery device with the body shell.

In an embodiment of the present application, the first avoidance hole is provided with the mounting buckle to realize the detachable mounting between the output end of the medicine delivery device and the main body shell.

In one implementation of an embodiment of the present application, the main body shell is provided with a slot; the baseplate is provided with an elastic fixing buckle; and
the slot is snap-fitted with the elastic fixing buckle so that the main body shell is mounted on the baseplate.

In an embodiment of the present application, the elastic fixing buckle is provided on the main body shell so that the main body shell and the medicine delivery device are facilitated to be securely and conveniently mounted on the baseplate.

In one implementation of an embodiment of the present application, the adhesive member is fixed to the baseplate by adhesive or ultrasonic welding.

In an embodiment of the present application, the adhesive member is fixed to the baseplate by adhesive or ultrasonic welding with a strong connection.

In one implementation of an embodiment of the present application, the medicine delivery device is detachably connected to the main body shell.

In an embodiment of the present application, the medicine delivery device is detachably connected to the main body shell to facilitate the replacement and refilling of medicinal liquid in the medicine delivery device.

In one implementation of an embodiment of the present application, the medicine delivery device is provided with a medicine reservoir, a connecting tube and a connecting needle sequentially;
the controlling device is used to pump out the medicinal liquid within the medicine reservoir so that the medicinal liquid flows from the medicine reservoir through the connecting tube to the connecting needle; the connecting needle is mounted on the first avoidance hole; and the connecting needle passes through the first avoidance hole to connect the injection device.

In an embodiment of the present application, the medicine delivery device is provided with a medicine reservoir, a connecting tube and a connecting needle. The connecting needle is mounted on the first avoidance hole and passes through the first avoidance hole to connect the injection device, achieving quick insertion and removal of the connecting needle to the injection device.

In one implementation of an embodiment of the present application, an integrated balanced paste-type medicine infusion system further includes a blood glucose meter; the blood glucose meter is communicatively connected to the controlling device; and the controlling device is used to control the medicine delivery device to output the medicinal liquid according to blood glucose data sent by the blood glucose meter. In an embodiment of the present application, the use of a blood glucose meter allows for effective monitoring of blood glucose levels and accurate control of medication delivery.

### Brief description of the drawings

Fig. 1 is a perspective view of the integrated balanced paste-type medicine infusion system of an embodiment in the present application;
Fig. 2 is another perspective view of the integrated balanced paste-type medicine infusion system of an embodiment in the present application;
Fig. 3 is a perspective view of the baseplate of the integrated balanced paste-type medicine infusion system of an embodiment in the present application;
Fig. 4 is a perspective view of the medicine delivery device and the main body shell of the integrated balanced paste-type medicine infusion system of an embodiment in the present application;
Fig. 5 is a perspective view of the medicine delivery device of the integrated balanced paste-type medicine infusion system of an embodiment in the present application;
Fig. 6 is another perspective view of the medicine delivery device of the integrated balanced paste-type medicine infusion system of an embodiment in the present application;
Fig. 7 is a perspective view of the main body shell of the integrated balanced paste-type medicine infusion system of an embodiment in the present application;
Fig. 8 is another perspective view of the main body shell of the integrated balanced paste-type medicine infusion system of an embodiment in the present application;
Fig. 9 is a diagram in a front view of the main body shell of the integrated balanced paste-type medicine infusion system of an embodiment in the present application;

1 injection device; 101 indwelling needle; 2 medicine delivery device; 201 medicine reservoir; 202 connecting tube; 203 connecting needle; 3 baseplate; 301 second avoidance hole; 302 welding point; 4 main body shell; 401 first avoidance hole; 402 mounting buckle; 403 elastic fixing buckle.

### Detailed description of the preferred embodiments

The terms "first", "second", "third", "fourth" and the like in the specification, the claims and the above-mentioned drawings of the present application are used to identify different objects and are not intended to describe a particular sequence or order. It should be understood that the ordinal numbers used in such a way are in appropriate cases interchangeable so that the embodiment described herein may be implemented in an order other than that illustrated or described herein. Additionally, the terms "comprise" and "include", and derivatives thereof, are intended to be interpreted non-exclusively. For example, a process, method, system, product or apparatus that includes a series of steps or units is not necessarily limited to those that are clearly listed, but may include other steps or units that are not clearly listed or that are inherent to the process, method, product or apparatus.

A portable medicine infusion system includes an external-type medicine infusion system and a paste-type medicine infusion system. There are problems with the external-type medicine infusion system such as relatively large size of the entire unit, entanglement and clogging of catheters, and large skin wound. The paste-type medicine infusion system is small and portable, which may be attached to the skin and be moved with humans or other living organisms.

The paste-type medicine infusion system is used to infuse the medicinal liquid into humans or other living organisms conveniently and continuously, which may also be used to infuse vary medicinal liquid according to the disease to be treated. The most common application scenario of the paste-type medicine infusion system is in the treatment of diabetic patients. Insulin is stored in the paste-type medicine infusion system for 24-hour management of the glucose level in diabetic patients. The following is an example of the paste-type medicine infusion system for the infusion of insulin to human diabetic patients, and such a paste-type medicine infusion system may also be referred to as a paste-type insulin pump.

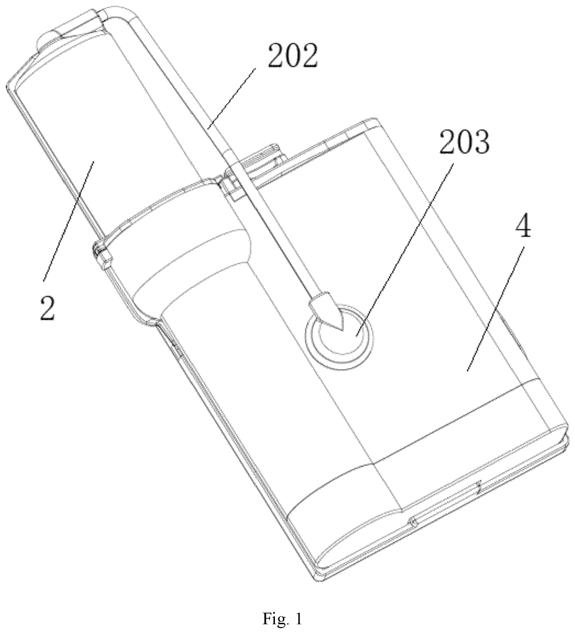

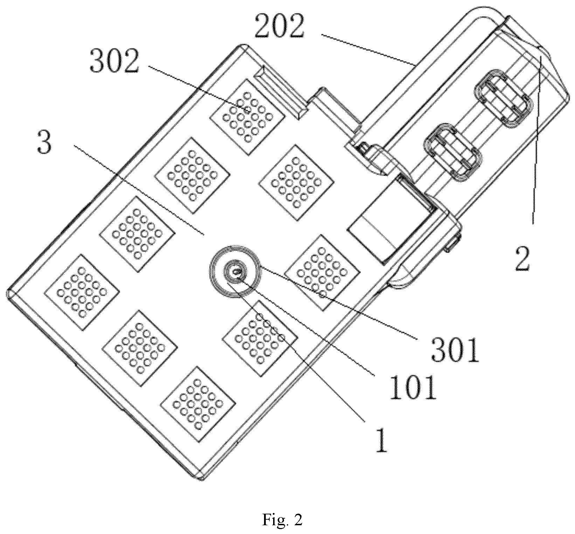

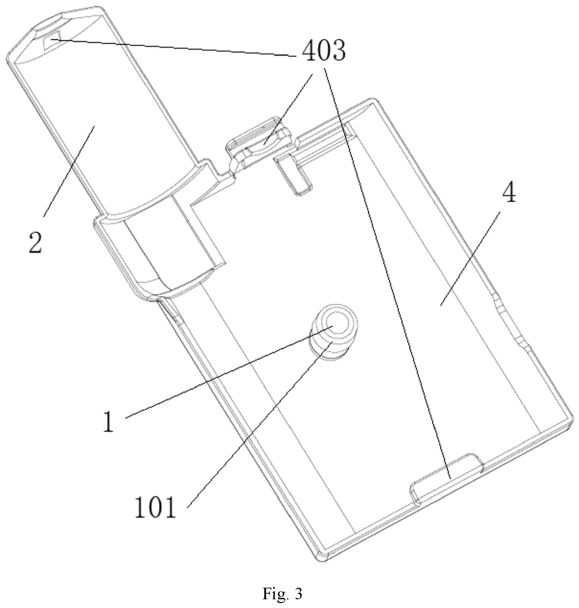

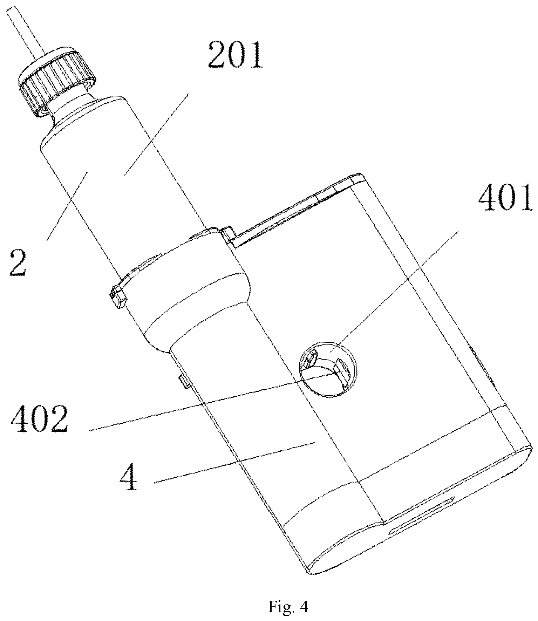

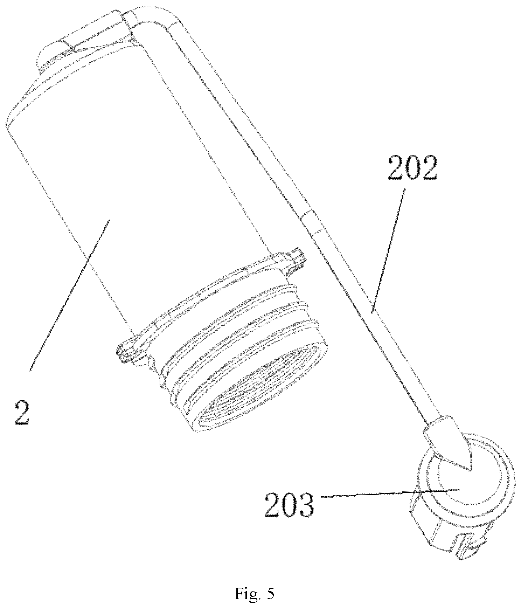

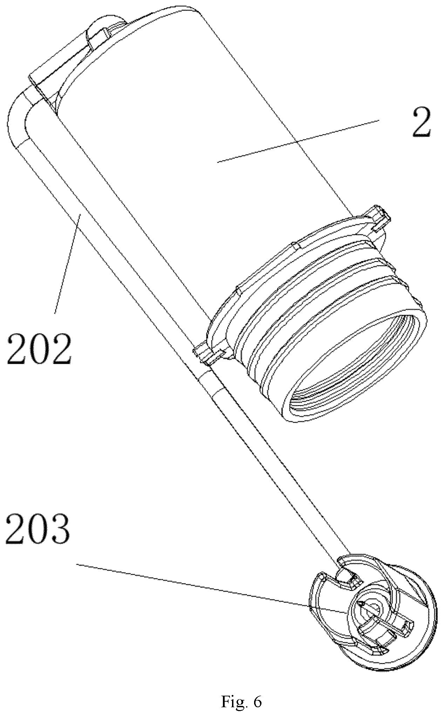

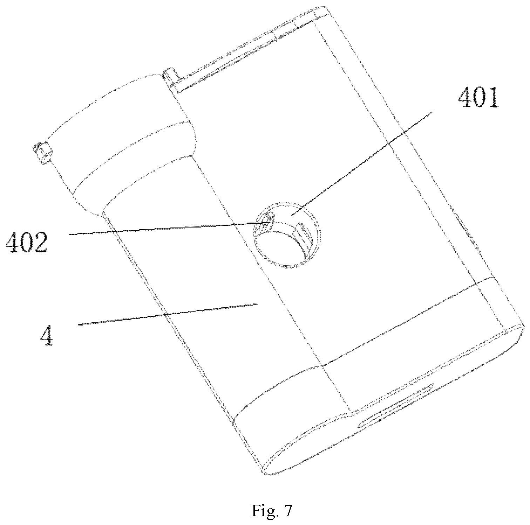

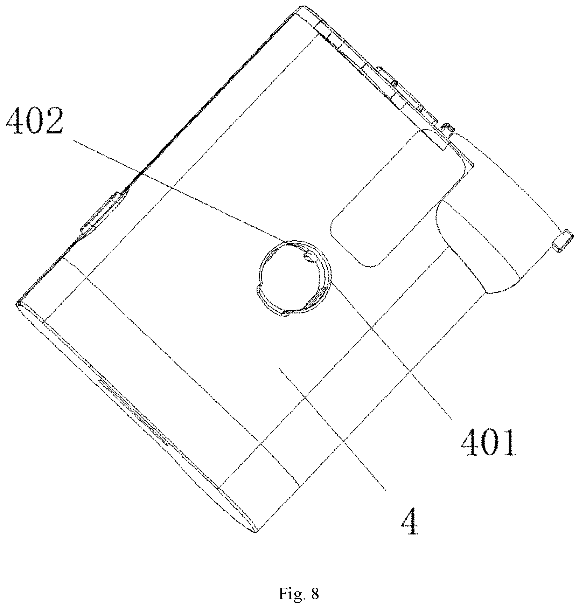

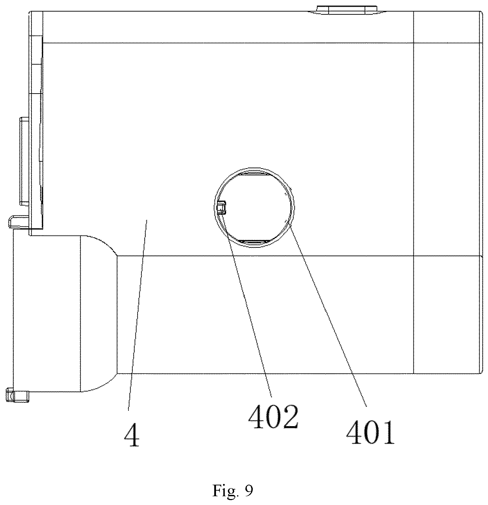

Referring to Fig. 1- Fig. 9, provided in the present application is an integrated balanced paste-type medicine infusion system including an injection device 1, a medicine delivery device 2, a baseplate 3, an adhesive member, a main body shell 4 and a controlling device;
the controlling device is mounted in the main body shell 4; the injection device 1, the medicine delivery device 2 and the main body shell 4 are mounted on the baseplate 3; the adhesive member is fixedly provided on a side of the baseplate 3 distal to the main body shell 4; then the adhesive member is used to attach the baseplate 3 to skin;
the medicine delivery device 2 is used to store medicinal liquid; an output end of the medicine delivery device 2 is connected to an input end of the injection device 1; the controlling device is used to control the medicine delivery device 2 to output the medicinal liquid to the injection device 1;
the main body shell 4 is provided with a first avoidance hole 401 penetratingly; the output end of the medicine delivery device 2 is mounted on the first avoidance hole 401;
the baseplate 3 is provided with a second avoidance hole 301 penetratingly; the injection device 1 is mounted on the second avoidance hole 301; and the second avoidance hole 301 is fitted with the first avoidance hole 401.

It is to be understood that the controlling device is mounted in the main body shell 4, the injection device 1 is mounted on the baseplate 3, and the medicine delivery device 2 is detachable mounted to the main body shell 4, which facilitates replacement. The medicine delivery device 2 is mounted first on the main body shell 4. Then, they are mounted on the baseplate 3 collectively. Finally, the output end of the medicine delivery device 2 is inserted into the injection device 1 through the first avoidance hole 401, in which the injection device 1 is mounted in the second avoidance hole 301 of the baseplate 3, achieving an integrated design. The baseplate 3 is used to attach the integrated balanced paste-type medicine infusion system to the skin.

In an embodiment of the present application, the main body shell 4 is provided with a first avoidance hole 401 penetratingly; the baseplate 3 is provided with a second avoidance hole 301 penetratingly; and the second avoidance hole 301 is fitted with the first avoidance hole 401, so that the first avoidance hole 401 is in communication with the second avoidance hole 301 in the state of use. The output end of the medicine delivery device 2 is mounted in the first avoidance hole 401 and the injection device 1 is mounted in the second avoidance hole 301, the output end of the medicine delivery device 2 is connected to the injection device 1. The controlling device is mounted in the main body shell 4, while the medicine delivery device 2 and the injection device 1 are mounted out of the main body shell. When separating the medicine delivery device 2, the controlling device, and the injection device 1, it is unnecessary to disassemble the main body shell 4 and simple to operate.

In addition, it may not only address the problem of the large size of the whole machine structure, but also alleviate the pain of patients due to the expansion of the wound when using the medicine infusion system.

Referring to Fig. 1- Fig. 9, provided in the present application is an integrated balanced paste-type medicine infusion system, including an injection device 1, a medicine delivery device 2, an baseplate 3, a main body shell 4 and a controlling device.

The injection device 1 is provided with an indwelling needle 101 and a needle holder. The needle holder is provided with a flexible sealing member to facilitate the output end of the medicine delivery device 2 to puncture the flexible sealing member for medicinal liquid infusion. The flexible sealing member may be a rubber member. The indwelling needle 101 may be a vertical indwelling needle 101.

The medicine delivery device 2 is provided with a medicine reservoir 201, a connecting tube 202 and a connecting needle 203 sequentially. The medicine delivery device 2 also includes a shell of the medicine delivery device 2, which is cylindrical in shape. An end of the shell of the medicine delivery device 2 is provided with an external thread for engaged with an internal thread on the main body shell 4. It is to be noted that the shell of the medicine delivery device 2 may also be detachably connected to the main body shell 4 by means of a buckle. The flexible sealing member may be provided on a side close to the main body shell 4 so as to facilitate the connecting needle 203 to be inserted into the first avoidance hole 401 and to puncture the flexible sealing member.

In the present embodiment, the integrated balanced paste-type medicine infusion system further includes a power supply device. The controlling device and the power supply device mounted within the main body shell 4. It is to be noted that the main body shell 4 may be replaced without disassembly of the power supply device. The power supply device may be a battery. The main body shell 4 may also be provided with a charging port for charging the power supply device. The power supply device is used to provide electrical energy to the medicine delivery device 2 and the controlling device.

In the present embodiment, the integrated balanced paste-type medicine infusion system further includes an adhesive member. The adhesive member fixed to the side of the baseplate 3 distal to the main body shell 4 by ultrasonic welding. The baseplate 3 is provided with welding points 302. The welding points 302 are used for connecting with the adhesive member. It is to be noted that the adhesive member may also be fixed on a side of the baseplate 3 distal to the main body shell 4 by adhesive. The adhesive member may be an adhesive tape.

The output end of the medicine delivery device 2 is a connecting needle 203. The connecting needle 203 is able to puncture the flexible sealing member. The medicine delivery device 2 is provided with a medicine reservoir for storing medicinal liquid. The controlling device sends a medicine delivery instruction to pump a certain amount of medicinal liquid from the medicine reservoir 201 at a certain speed. The medicinal liquid flows out of the medicine reservoir 201 and then flows sequentially through the connecting tube 202, the connecting needle 203 and the indwelling needle 101, and ultimately flows into the body of humans or other living organisms. A driving main unit provided in the controlling device may be a motor or an electric pump.

The controlling device may be in a timing mode or in a monitoring mode. In the timing mode, the controlling device sends a medicine delivery instruction to the driving main unit based on a preset medicine delivery time, so as to drive the driving main unit to complete delivery of the medicinal liquid based on the preset medicine delivery time. In the monitoring mode, in the present embodiment, the integrated balanced paste-type medicine infusion system further includes a blood glucose meter. The blood glucose meter detects the blood glucose of humans or other organisms to obtain blood glucose data, and sends the blood glucose data to the controlling device. The controlling device receives the blood glucose data sent by the blood glucose meter, and determines whether the current blood glucose value is larger than a preset value. Sending a medicine delivery instruction to the driving main unit if the current blood glucose value is larger than the preset value. Not sending a medicine delivery instruction to the driving main unit if the current blood glucose value is not larger than the preset value. The blood glucose meter may communicate with the controlling device via Bluetooth, etc.

In the present embodiment, the main body shell 4 is provided with a first avoidance hole 401 and the baseplate 3 is provided with the second avoidance hole 301. When the main body shell 4 is mounted on the baseplate 3, the first avoidance hole 401 is in communication with the second avoidance hole 301. The first avoidance hole 401 and the second avoidance hole 301 may be round in shape.

A mounting buckle 402 is provided in the first avoidance hole 401, and the connecting needle 203 of the medicine delivery device 2 is snap-fitted into the first avoidance hole 401 by the mounting buckle 402. The injection device 1 is snap-fitted to the second avoidance hole 301 by the needle holder. The injection device 1 is inserted into the first avoidance hole 401 from a side of the main body shell 4 close to the baseplate 3, and the connecting needle 203 is inserted into the first avoidance hole 401 from a side of the main body shell 4 distal to the baseplate 3. The connecting needle 203 is connected to the injection device 1 through the first avoidance hole 401, and the connecting needle 203 punctures the flexible sealing member for medicine infusion. The connecting needle 203 is provided with a connecting buckle to match the mounting buckle 402 in the first avoidance hole 401, and the mounting buckle 402 and the connecting buckle are detachably connected.

The second avoidance hole 301 is provided in a central predetermined area of the baseplate 3. A distance between a boundary of the central predetermined area of the baseplate 3 and an edge of the baseplate 3 is larger than a first preset distance, in which the first preset distance may be such as 1cm, 2cm, and 3cm. Alternatively, a distance between the geometrical center of the central predetermined area of the baseplate 3 and the geometrical center of the baseplate 3 is smaller than a second predetermined distance, in which the second preset distance may be such as 1cm, 2cm, and 3cm.

Since the second avoidance hole 301 is fitted with the first avoidance hole 401, the first avoidance hole 401 is provided in the central predetermined area of the main body shell 4, and the position of the first avoidance hole 401 may be determined in accordance with the position of the second avoidance hole 301 and the mounting position between the baseplate 3 and the main body shell 4, realizing a balanced design.

The main body shell 4 is provided with an elastic fixing buckle 403, and the baseplate 3 is snap-fitted to the elastic fixing buckle 403 so that the controlling device is mounted on the baseplate 3. It is to be noted that the shell of the medicine delivery device 2 may also be provided with an elastic fixing buckle 403, enabling the controlling device and the delivery device 2 to be mounted on the baseplate 3.

Referring to Fig. 1- Fig. 9, provided in the embodiment of the present application is an integrated balanced paste-type medicine infusion system, including an injection device 1, a medicine delivery device 2, a baseplate 3, an adhesive member, a main body shell 4 and a controlling device;
the controlling device is mounted in the main body shell 4; the injection device 1, the medicine delivery device 2 and the main body shell 4 are mounted on the baseplate 3; the adhesive member is fixedly provided on a side of the baseplate 3 distal to the main body shell 4; then the adhesive member is used to attach the baseplate 3 to skin;
the medicine delivery device 2 is used to store medicinal liquid; an output end of the medicine delivery device 2 is connected to an input end of the injection device 1; the controlling device is used to control the medicine delivery device 2 to output the medicinal liquid to the injection device 1;
the main body shell 4 is provided with a first avoidance hole 401 penetratingly; the output end of the medicine delivery device 2 is mounted on the first avoidance hole 401;
the baseplate 3 is provided with a second avoidance hole 301 penetratingly; the injection device 1 is mounted on the second avoidance hole 301; and the second avoidance hole 301 is fitted with the first avoidance hole 401.

The second avoidance hole 301 is provided in a central predetermined area of the baseplate 3, so that the injection device 1 is provided in a central position of the entire integrated balanced paste-type medicine infusion system.

The output end of the medicine delivery device 2 is inserted into the first avoidance hole 401 from a side of the main body shell 4 distal to the baseplate 3; the injection device 1 is mounted in the second avoidance hole 301 of the baseplate 3; and the injection device 1 is inserted into the first avoidance hole 401 from a side of the main body shell 4 proximal to the baseplate 3.

The injection device 1 includes an indwelling needle 101 and a needle holder, and the needle holder is used to mount the indwelling needle 101 to the baseplate 3.

The main body shell 4 is provided with a mounting buckle 402 in the first avoidance hole 401, and the mounting buckle 402 is used to snap-fit the output end of the medicine delivery device 2 with the body shell 4.

The main body shell 4 is provided with a slot; the baseplate 3 is provided with an elastic fixing buckle 403; and

the slot is snap-fitted with the elastic fixing buckle 403 so that the main body shell 4 is mounted on the baseplate 3.

The adhesive member is fixed to the baseplate 3 by ultrasonic welding. A plurality of welding points 302 are provided on a side of the baseplate 3 distal to the main body shell 4. Alternatively, the adhesive member is fixed to the baseplate 3 by adhesive.

The medicine delivery device 2 is detachably connected to the main body shell 4.

The medicine delivery device 2 is provided with a connected of a medicine reservoir 201, a connecting tube 202, and a connecting needle 203 sequentially;
the controlling device is used to pump out the medicinal liquid within the medicine reservoir 201 so that the medicinal liquid flows from the medicine reservoir 201 through the connecting tube 202 to the connecting needle 203; the connecting needle 203 is mounted on the first avoidance hole 401; and the connecting needle 203 passes through the first avoidance hole 401 to connect the injection device 1.

The integrated balanced paste-type medicine infusion system further includes a blood glucose meter; the blood glucose meter communicatively connected to the controlling device; and the controlling device is used to control the medicine delivery device to 2 output the medicinal liquid based on blood glucose data sent by the blood glucose meter.

Referring to Fig. 1- Fig. 9, provided in the present embodiment of the application is an integrated balanced paste-type medicine infusion system, including a controlling device, a medicine delivery device 2, an injection device, a baseplate 3 and an adhesive tape. The injection device 1 may be referred to as an indwelling needle assembly; the indwelling needle assembly includes an indwelling needle 101 and a needle holder; and the indwelling needle assembly is positioned in a center of the baseplate 3. The adhesive tape is attached to the baseplate 3, and the fixation method therebetween may be ultrasonic welding or adhesive. The controlling device is fixed to the baseplate 3 by means of the elastic fixing buckle 403. The controlling device is threaded to the medicine delivery device 2, an end of the medicine delivery device 2 is provided with a connecting needle 203. The connecting needle 203 passes through the avoidance positioning design of the controlling device, and punctures the rubber member at the top of the injection device 1 fixed to the baseplate 3, and conducts with the internal channel of the injection device 1. The main body shell 4 provided with the controlling device includes a top cover and a bottom cover. The top cover is concave to form a chamber, and the bottom cover is covered on the opening of the top cover to form a sealed space with the top cover, in which the driving main unit, the pusher, the battery, and other controlling devices are all provided therein. The driving main unit drives the pusher to move and reduce the volume of the medicine reservoir 201, so that the medicinal liquid is pumped out from the medicine reservoir 201. The medicine infusion system adopting a paste-style design in the present embodiment is directly attached to the user's abdomen, limbs and other locations, without additional connection to a catheter, which may be directly attached to the skin for use, omitting a long catheter, reducing the volume, and increasing portability, safety, and the comfort of wearing the device. In the present embodiment, the injection device 1, the medicine delivery device 2 and the controlling device are designed as a separable type, and the controlling device may be used repeatedly, which reduces waste and saves costs.

Finally, it should be noted that the above embodiments are only used to illustrate the technical solutions of the present application, but not to limit them. Although the present application has been described in detail with reference to the above embodiments, those skilled in the art should understand that the technical solutions recorded in the above embodiments may still be modified, or some of the technical features may be substituted equivalently. These modifications or substitutions do not take the essence of the corresponding technical solutions out of the spirit and scope of the technical solutions of various embodiments of the present application.

## Claims

1. An integrated balanced paste-type medicine infusion system, comprising an injection device (1), a medicine delivery device (2), a baseplate (3), an adhesive member, a main body shell (4) and a controlling device;
the controlling device is mounted in the main body shell (4); the injection device (1), the medicine delivery device (2) and the main body shell (4) are mounted on the baseplate (3); the adhesive member is fixedly provided on a side of the baseplate (3) distal to the main body shell (4); then the adhesive member is used to attach the baseplate (3) to skin;
the medicine delivery device (2) is used to store medicinal liquid; an output end of the medicine delivery device (2) is connected to an input end of the injection device (1); the controlling device is used to control the medicine delivery device (2) to output the medicinal liquid to the injection device (1);
the main body shell (4) is provided with a first avoidance hole (401) penetratingly; the output end of the medicine delivery device (2) is mounted on the first avoidance hole (401);
the baseplate (3) is provided with a second avoidance hole (301) penetratingly; the injection device (1) is mounted on the second avoidance hole (301); and the second avoidance hole (301) is in communication with the first avoidance hole (401).

2. The integrated balanced paste-type medicine infusion system according to claim 1, wherein the second avoidance hole (301) is provided in a central predetermined area of the baseplate (3), so that the injection device (1) is provided in a central position of the entire integrated balanced paste-type medicine infusion system.

3. The integrated balanced paste-type medicine infusion system according to claim 1 or 2, wherein the output end of the medicine delivery device (2) is inserted into the first avoidance hole (401) from a side of the main body shell (4) distal to the baseplate (3); the injection device (1) is mounted in the second avoidance hole (301) of the baseplate (3); and the injection device (1) is inserted into the first avoidance hole (401) from a side of the main body shell (4) proximal to the baseplate (3).

4. The integrated balanced paste-type medicine infusion system according to claim 3, wherein the injection device (1) comprises an indwelling needle (101) and a needle holder, and the needle holder is used to mount the indwelling needle (101) to the baseplate (3).

5. The integrated balanced paste-type medicine infusion system according to claim 4, wherein the main body shell (4) is provided with a mounting buckle (402) in the first avoidance hole (401), and the mounting buckle (402) is used to snap-fit the output end of the medicine delivery device (2) with the body shell (4).

6. The integrated balanced paste-type medicine infusion system according to claim 1, 2, 4 or 5, wherein the main body shell (4) is provided with a slot; the baseplate (3) is provided with an elastic fixing buckle (403); and
the slot is snap-fitted with the elastic fixing buckle (403) so that the main body shell (4) is mounted on the baseplate (3).

7. The integrated balanced paste-type medicine infusion system according to claim 6, wherein the adhesive member is fixed to the baseplate (3) by adhesive or ultrasonic welding.

8. The integrated balanced paste-type medicine infusion system according to claim 1, 2, 4, 5 or 7, wherein the medicine delivery device (2) is detachably connected to the main body shell (4).

9. The integrated balanced paste-type medicine infusion system according to claim 8, wherein the medicine delivery device (2) is provided with a medicine reservoir (201), a connecting tube (202) and a connecting needle (203) sequentially;
the controlling device is used to pump out the medicinal liquid within the medicine reservoir (201) so that the medicinal liquid flows from the medicine reservoir (201) through the connecting tube (202) to the connecting needle (203); the connecting needle (203) is mounted on the first avoidance hole (401); and the connecting needle (203) passes through the first avoidance hole (401) to connect the injection device (1).

10. The integrated balanced paste-type medicine infusion system according to claim 1, 2, 4, 5, 7 or 9, comprising a blood glucose meter; the blood glucose meter is communicatively connected to the controlling device; and the controlling device is used to control the medicine delivery device (2) to output the medicinal liquid according to blood glucose data sent by the blood glucose meter.
